# EUROPEAN PATENT APPLICATION

(11) **EP 3 578 180 A1**
(43) Date of publication of application: **11.12.2019**
(21) Application number: 18176120.6
(22) Date of filing: 05.06.2018
(51) Int. Cl.: A61K 31/4412, A61K 45/06, A61K 31/16, A61P 9/10

(54) **MODULATORS OF SERYL-TRNA SYNTHASE AND PHARMACEUTICAL COMPOSITIONS COMPRISING THE SAME FOR INCREASING CELL HYPOXIC TOLERANCE**

(71) Applicant: Centre National de la Recherche Scientifique (CNRS), 75794 Paris Cedex 16 (FR); Université Nice Sophia Antipolis (UNS), 06100 Nice (FR)
(72) Inventor: TAUC, Michel, 06000 NICE (FR); COUGNON, Marc, 06200 NICE (FR); DURANTON, Christophe, 06100 NICE (FR); BLONDEAU, Nicolas, 06000 NICE (FR); RUBERA, Isabelle, 06100 NICE (FR)
(74) Representative: Macquet, Christophe

(57) **Abstract**

The invention relates to a seryl-tRNA synthetase modulator for use in preventing and/or treating disorders related to hypoxic conditions and/or ischemia injury in a mammal in a need thereof. The invention also concerns a pharmaceutical composition for use in preventing and/or treating disorders related to hypoxic conditions and/or ischemia injury in a mammal in a need thereof, said composition comprising a seryl-tRNA synthetase modulator and a pharmaceutically acceptable carrier.

SEQUENCE LISTING

<110>  Centre National de la recherche Scientifique (CNRS)
       Université Nice Sophia Antipolis (UNS)

<120>  MODULATORS AND PHARMACEUTICAL COMPOSITIONS COMPRISING THE SAME
       FOR INCREASING CELL HYPOXIC TOLERANCE

<130>  11054EP

<160>  4

<170>  PatentIn version 3.5

<210>  1
<211>  20
<212>  DNA
<213>  Homo sapiens

<400>  1
gtcggggcta cactccaatc                                                   20


<210>  2
<211>  20
<212>  DNA
<213>  Homo sapiens

<400>  2
tttcgctgcc tttgccaatc                                                   20


<210>  3
<211>  19
<212>  DNA
<213>  Homo sapiens

<400>  3
gctggtgaaa aggacctct                                                    19


<210>  4
<211>  20
<212>  DNA
<213>  Homo sapiens

<400>  4
cacaggacta gaacacctgc                                                   20

## Description

### TECHNICAL FIELD

The present invention aims to provide new treatments for helping patients who suffer from disorders related to hypoxic conditions and/or ischemia injury. The invention relates to a new modulator for use in preventing and/or treating disorders related to hypoxic conditions and/or ischemia injury in a mammal in a need thereof. The invention also concern pharmaceutical composition comprising the same for use in preventing and/or treating disorders related to hypoxic conditions and/or ischemia injury in a mammal in a need thereof.

### BACKGROUND OF THE INVENTION

Hypoxia, anoxia or ischemia is a condition in which the body, a region of the body or an organ is deprived of adequate oxygen supply at the tissue level. It may happen from various ways: firstly when healthy people climb to higher altitude (also called natural hypoxia) and on the other hand due to an ischemic injury (also called pathologic hypoxia). Hypoxia result more precisely from a failure at any stage in the delivery of oxygen to cells. This can include decreased partial pressures of oxygen, problems with diffusion of oxygen in the lungs, insufficient available hemoglobin, problems with blood flow to the end tissue, and problems with breathing rhythm. These disorders can lead to an arrest of the tissue or cell function. Different causes induce hypoxia, which may be due for example to:
- the presence of a blood clot obstructing an artery (thrombosis);
- the formation of an atheromatous plaque;
- the hemorrhage or hypo-perfusion preventing certain tissues from being properly fed;
- the artery compression by an external object/events, or by an internal phenomenon; and
- a surgical procedure requiring a blood flow arrest such as aortic surgery or organ transplantation.

Depending on the duration of ischemia its consequences are gradual from transitory (reversible) to irreversible leading to the organ infarct. The molecular mechanisms responsible of tissue damages are well known, and comprise:
- a cellular acidosis and consequences of intracellular pH deregulation on intracellular sodium and calcium homeostasis;
- metabolic disturbances, reduction of ATP stocks and activation of AMP kinase;
- inability to maintain cell membrane potential, necrosis;
- mitochondrial damage, production of free radicals; and
- apoptosis.

Preventing the consequences of hypoxia/ischemia is a major therapeutic goal in human health. Ischemic events resulting from various pathological episodes are among the most deleterious clinical situation especially when occurring in highly metabolically active tissues like the heart and brain. While many heart attack treatments have been developed, there are no efficacious pharmacological treatments to reduce brain injury and/or improve long-term outcomes for patients suffering from stroke (brain attack). Ischemic stroke that accounts for about 85% of all cases of brain ischemia remains a leading cause of human disability with long-term motor and cognitive deficits characterized by aphasia, slowed information processing and executive dysfunction.

Several pharmaceutical research and clinical trials focused on preventing ischemic injury. As an example, WO2012/010641 A2 discloses a new class of cytoprotective agents that allows to an enhancing of the global survival rate of mammal cells subjected to hypoxic conditions, and to prevent the disorders related to an oxygen deprivation. However, the cytoprotective agents disclosed in this document seem to be not really specific, and improvements are still needed.

Based on the above, and in the context of the present invention, there remains a need for identifying new targets and new associated cytoprotective agents that can be used as new specific drugs to limit the consequences of cell, tissue or organ ischemia. In particular, there remains a need to increase cellular hypoxic tolerance in a mammal subjected to hypoxic conditions and/or ischemia injury.

### SUMMARY OF THE INVENTION

In accordance with a first aspect, the invention relates to a seryl-tRNA synthetase (SerRS) modulator for use in preventing and/or treating disorders related to hypoxic conditions and/or ischemia injury in a mammal in a need thereof.

According to a second aspect, the invention concerns a pharmaceutical composition for use in preventing and/or treating disorders related to hypoxic conditions and/or ischemia injury in a mammal in a need thereof, said composition comprising at least one seryl-tRNA synthetase modulator and a pharmaceutically acceptable carrier.

In a third aspect, the invention relates the use of a seryl-tRNA synthetase modulator, or a pharmaceutical composition comprising at least one seryl-tRNA synthetase modulator and a pharmaceutically acceptable carrieras a cytoprotective agent in an ex *vivo* organ perfusion and preservation fluid.

In a fourth aspect, the invention relates to a a seryl-tRNA synthetase modulator or to a pharmaceutical composition comprising at least one seryl-tRNA synthetase modulator and a pharmaceutically acceptable carrier, for reducing the oxygen demand of a mammal, preferably a human.

In a fifth aspect, the invention relates to a liquid for ex *vivo* organ preservation comprising a seryl-tRNA synthetase modulator or a pharmaceutical composition comprising at least one seryl-tRNA synthetase modulator and a pharmaceutically acceptable carrier.

In a sixth aspect, the invention concerns a method for preserving or treating organs in their viable states in brain-dead patients or cadavers, wherein a seryl-tRNA synthetase modulator or a pharmaceutical composition comprising at least one seryl-tRNA synthetase modulator and a pharmaceutically acceptable carrier, is a administered to the brain-dead patient or the cadaver.

In a seventh aspect, the invention relates to a method for preserving or treating an harvested organ in their viable states wherein a seryl-tRNA synthetase modulator or a pharmaceutical composition comprising at least one seryl-tRNA synthetase modulator and a pharmaceutically acceptable carrier, is administered by perfusion to said organ.

Advantageously, the seryl-tRNA synthetase modulator for use in preventing and/or treating disorders related to hypoxic conditions and/or ischemia injury in a mammal in a need thereof is characterized in that: - the modulator is an inhibitor; - the disorders related to hypoxic conditions are selected from the group consisting of kidney failure, heart failure, neurological disorders, stroke, heart attack, atrial fibrillation, angiopathy, peripheral and central microangiopathy, thrombosis, infarction, pulmonary embolism, ischemic colitis and mesenteric ischemia, and organ dysfunction following a transplantation procedure said organs being selected from liver, bowel, heart, and lung; - the modulator is selected from the group consisting of serine hydroxamate, serine analogs, non-metabolisable serine derivatives, compound SB-217452, molecules able to specifically bind seryl tRNA synthetase in a competitive, uncompetitive, or noncompetitive way and siRNA targeting seryl-tRNA synthetase gene; - the modulator is administered for enhancing hypoxia tolerance of mammal cells subject to hypoxic conditions and/or ischemia injury; - the seryl-tRNA synthetase is in its mitochondrial form or its cytoplasmic form, preferably in its cytoplasmic form; - it is administered by parenteral delivery and enteral delivery including per os, nasogastric, percutaneous endoscopic gastrostomy tubing, aerosol, intraperitoneal and intravenous route; - it is administered by one single injection, by sequential injections or by continuous infusion preceding or following the disorders related to hypoxic conditions and/or ischemia injury.

Advantageously, the pharmaceutical composition for use in preventing and/or treating disorders related to hypoxic conditions and/or ischemia injury in a mammal in a need thereof is characterized in that: - the composition is administered by parenteral delivery and enteral delivery including, per os, nasogastric, percutaneous endoscopic gastrostomy tubing, aerosol, intraperitoneal and intravenous route; - it is administered by one single injection, by sequential injections or by continuous infusion preceding or following the disorders related to hypoxic conditions and/or ischemia injury.

### BRIEF DESCRIPTION OF DRAWINGS

Other features and aspects of the present invention will be apparent from the following description and the accompanying drawings, in which:
Fig.1A illustrates the quantification of kidney cells viability under anoxia conditions, wherein said cells are prior subjected to a global translational inhibitor (CHX) and to a specific inhibitor of seryl-tRNA synthetase (SHX);
Fig.1B illustrates representative fluorescent images of Proximal Convoluted Tubule cells (PCT) wherein cells are exposed to normoxia or anoxia conditions, and wherein the cycloheximide (CHX) and the serine hydroxamate (SHX) are evaluated for cell protection;
Fig.2A illustrates mRNA expression levels of the serRS gene in control cells and in cells transfected with non-targeting (siScramble) or serRS pooled siRNAs;
Fig.2B illustrates the quantification of kidney cells viability under anoxia conditions after mRNA silencing of serRS;
Fig.2C illustrates a representative fluorescent images of PCT kidney cells exposed to normoxia or anoxia conditions and which are transfected with serRS or non-targeting control pooled siRNAs;
Fig.3A illustrates a quantification of cell death in HT22 neuronal cells subjected to an Oygen Glucose Deprivation (OGD), wherein serine hydroxamate (SerHX) is evaluated for cell protection;
Fig.3B illustrates a representative cresyl violet staining showing the protective effect of intraperitoneal (IP) injections or a single intravenous (IV) injection of serine hydroxamate (SerHX) before intraluminal occlusion of the right middle cerebral artery in mice (tMCAo);
Fig.3C illustrates a quantitation of the infarct volume, wherein the ischemic lesion is reduced by SerHX pretreatment;
Fig.4A illustrates the Rotarod test performed on mice, which has suffered from a stroke, for detecting the effect of SerHX post-treatment on motor deficits;
Fig.4B illustrates the long-term survival rate of mice after 30 min of tMCAo;
Fig.4C illustrates the latency to find the hidden platform during the post-stroke learning period in the Morris Water-Maze test;
Fig.4D illustrates the time spent by mice in the target zone (where the platform was previously located before its removal) in the Morris Water-Maze test; and
Fig.4E illustrates a representative searching behavior pattern of a mice 18 days post-stroke.

### DETAILLED DESCRIPTION OF THE INVENTION

The invention relates to a seryl-tRNA synthetase modulator for use in preventing and/or treating disorders related to hypoxic conditions and/or ischemia injury in a mammal in a need thereof.

According to the invention, the mammal is in particular a human. However, all mammals are concerned including nonhuman primate; pets like cat and dog; domesticated ungulates like horse and cow; or rodents such as mice and rats.

As used herein, the terms "hypoxic conditions" refer to conditions wherein the cells/tissues/organs have a decrease in oxygen supply as compared to standard conditions. In particular, in the present invention, the "hypoxic conditions" mean that the oxygen supply is reduced by 30%, preferentially by 50%, even more preferentially by 80% and in certain cases by 100%. The oxygen level can be easily measured by determining the blood oxygen level in a mammals in a need thereof, preferably a human, and comparing such a level to a standard value taken in a mammal in good health, preferably a human.

The terms "ischemia" or "hypoxia" refer to a situation wherein cell/tissue/organ or even a mammal, preferably a human, is partially or totally oxygen-deprived, such as for example when the bloodstream is clamped or cut, or when a mammal is in an unventilated area. After a stroke, every minute counts because harmful changes to the brain's synaptic connections occur within the first 3 minutes following a stroke. In particular, ischemia is also accompanied by glucose and energy metabolites deprivations which are contained in blood. The term "anoxia" refers to a situation wherein cell/tissue/organ or even a mammal, preferably a human, is totally oxygen-deprived.

As used herein, the term "modulator" refers to a compound that alters an activity of a molecule. For example, a modulator can cause an increase or a decrease in the magnitude of a certain activity of a molecule compared to the magnitude of the activity in the absence of the modulator. In certain embodiments, a modulator is an inhibitor, which decrease the magnitude of one or more activities of a molecule. In certain embodiments, an inhibitor completely prevents one or more activities of a molecule. As used herein, the term "inhibitor" refers to a biologic or a chemical synthesis compound which reduces or blocks the overall activity of an enzyme. An inhibitor can act by preventing the binding of the enzyme natural substrate (competitive inhibition), binding to the complex formed between the enzyme and the substrate (uncompetitive inhibition) or both (noncompetitive inhibition). It can bind reversibly or irreversibly. An inhibitor can modify one or more sites on or near the active site of the enzyme, or it can cause a conformational change elsewhere on the enzyme. In the context of the present invention, the term inhibitor also encompasses biological or chemical compounds that impact on the expression of the genes encoding for the enzyme. In another embodiments, a modulator is an activator, which increases the magnitude of at least one activity of a molecule. In certain embodiments, the presence of a modulator results in an activity that does not occur in the absence of the modulator.

In a preferred embodiment of the invention, the seryl-tRNA synthetase modulator is an seryl-tRNA synthetase inhibitor.

Seryl-tRNA synthetase is a protein that is involved in the protein synthesis. It belongs to the class II aminoacyl-tRNA synthetase family and is found in all humans. The seryl-tRNA synthetase is made up of 514 aminoacid residues, and it exists as a homodimer of two identical subunits, with the tRNA molecule binding across the dimer by similarity. Seryl-tRNA synthetase has two distinct domains (i) a catalytic core, and (ii) a 3 base pair serine binding n-terminal extension. The seryl-tRNA synthetase are involved in protein translation. Such a protein catalyzes in particular the transfer of L-serine to tRNA (Ser) . The cytosolic enzyme recognizes its cognate tRNA species and binds with a high level of specificity, allowing the accurate interaction between corresponding codons and anticodons on mRNA and tRNA during protein translation.

In the context of the invention, the seryl-tRNA synthetase can be in its mitochondrial form or in its cytoplasmic form, and preferably in its cytoplasmic form.

According to the present invention, the disorders related to hypoxic conditions are in particular selected from the group consisting of kidney failure, heart failure, neurological disorders such as for example headache, amyotrophic lateral sclerosis, arteriovenous malformation, brain aneurysm, brain tumors, dural arteriovenous fistulae, epilepsy, memory disorders, multiple sclerosis, Parkinson's disease, peripheral neuropathy, post-herpetic neuralgia and spinal cord tumor, stroke such as for example ischemic stroke, hemorrhagic stroke and transient ischemic attacks (TIAs), heart attack, atrial fibrillation, angiopathy, peripheral and central microangiopathy, thrombosis, infarction, pulmonary embolism, ischemic colitis and mesenteric ischemia, such as for example ischemia of the large and small bowel, respectively, and organ dysfunction following a transplantation procedure said organs are selected by liver, bowel, heart, and lung.

As used in the context of the invention, the terms "organ dysfunction subjected to hypoxia or hypoxic conditions" refer to molecular mechanisms related to hypoxia known by the ordinary skill in the art, and the consequences of such mechanisms on the general state of the organ (tissues damages, dysfunctions). As examples of consequences or dysfunctions, it is disclosed the kidney failure, the heart failure, the ischemic- and hypoxic-induced neurodegeneration and its neurologic consequences the organ dysfunctions or the tissue damages impacting liver, bowel, heart, lung, kidney, limbs or other organs following a transplantation procedure, or following climbing or even scuba diving. All cells types may be concerned and, in particular, cells which have a high oxygen demand.

According to a preferred embodiment of the invention, the seryl-tRNA synthetase modulator is selected from the group consisting of serine hydroxamate, cycloheximide, serine derivatives or serine analogs, non-metabolisable serine derivatives, the compound SB-217452 as described in Stefanska, A. L., M. Fulston, C. S. V. Houge-Frydrych, J. J. Jones, and S. R. Warr. 2000. "A potent seryl tRNA synthetase inhibitor SB-217452 isolated from a Streptomyces species". J. Antibiot. 53:1346-1353., molecules able to specifically bind seryl tRNA synthetase in a competitive, uncompetitive, or noncompetitive way and siRNA targeting the SerRS gene.

According to another preferred embodiment of the invention, the seryl-tRNA synthetase modulator is administered for enhancing hypoxia tolerance of mammal cells subject to hypoxic conditions and/or ischemia injury.

In a preferred embodiment, the seryl-tRNA synthetase modulator is administered by parenteral or enteral delivery. This includes per os (oral), nasogastric or percutaneous endoscopic gastrostomy tubing, aerosol or intraperitoneal and intravenous route, such as for example delivered via central or peripheral venous line, preferably by intravenous route. The latter could enable compound delivery by continuous infusion, if steady state dosing conditions are required on longer periods of treatment or in clinical setting.

In another preferred embodiment, the seryl-tRNA synthetase modulator is administered by one single injection, by sequential injections or by continuous infusion preceding and/or following the disorders related to hypoxic conditions and/or ischemia injury.

As used herein, the terms "sequential injections" refer to at least 2 injections, preferably at least 3 injections, and even more preferably at least 4 injections preceding or following the disorders related to hypoxic conditions and/or ischemia injury. For example, the first injection may occur 24h and the second injection may occur 2h preceding the disorders related to hypoxic conditions and/or ischemia injury. In another example, the first injection may occur 2h and the second injection may occur 24h following the disorders related to hypoxic conditions and/or ischemia injury. It is understand, that the ordinary skill in the art is able to adapt the number of injections and the frequency of the injections according to the nature and the clinical severity of the disorder.

According to a second aspect, the invention provides a pharmaceutical composition for use in preventing and/or in treating disorders related to hypoxic conditions and/or ischemia injury in a mammal in a need thereof. The pharmaceutical composition comprises at least one seryl-tRNA synthetase modulator and a pharmaceutically acceptable carrier. The seryl-tRNA synthetase modulator is one of the same that previously described in the specification. The seryl-tRNA synthetase modulator is, in a preferred embodiment, a seryl-tRNA synthetase inhibitor.

In a preferred embodiment, the pharmaceutical composition prevents or treats the same disorders related to hypoxic conditions that the ones previously described in the specification.

The pharmaceutical composition is in particular administered by parenteral or enteral delivery. This includes per os (oral), nasogastric or percutaneous endoscopic gastrostomy tubing, aerosol, or intraperitoneal and intravenous route, such as for example delivered via central or peripheral venous line, preferably by intravenous route. The latter could enable composition delivery by continuous infusion, if steady state dosing conditions are required on longer periods of treatment or in clinical setting. The pharmaceutical composition is particularly administered by sequential injections following the disorders related to hypoxic conditions and/or ischemia injury. In an another embodiment, the pharmaceutical composition is administered in a single injection. In an another embodiment, the pharmaceutical composition is administered by a continuous infusion.

In the context of the present invention, with a view to an organ transplantation such as liver, bowel, heart, lung or kidney, the time of ischemia from picking to transplantation is the main variable that is taken is account in the organ supply chain management from the organ initial location to its allocation and distribution. these organs are transported for a certain amount of time or distance long or short. In a general manner, the grafts are maintained at 4°c in an appropriated media adapted to each organ. The cold ischemia time of organ transplants is the time period during the graft is refrigerated. It has been demonstrated that the risk of failure of an organ transplantation increases with the increasing of the cold ischemia time. In such a case, the ischemic injury must not exceed few hours upon pain of irreversible damages.

According to a third aspect, the invention concerns a use of a seryl-tRNA synthetase modulator, or a pharmaceutical composition comprising at least one seryl-tRNA synthetase modulator and a pharmaceutically acceptable carrier as a cytoprotective agent in an *ex vivo* organ perfusion and preservation fluid.. The seryl-tRNA synthetase modulator is one of the same that previously described in the specification. The seryl-tRNA synthetase modulator is, in a preferred embodiment, a seryl-tRNA synthetase inhibitor. The pharmaceutical composition is one of the same that previously described in the specification.

In a fourth aspect, the invention relates to a a seryl-tRNA synthetase modulator or to a pharmaceutical composition comprising at least one seryl-tRNA synthetase modulator and a pharmaceutically acceptable carrier, for reducing the oxygen demand of a mammal, preferably a human. The seryl-tRNA synthetase modulator is one of the same that previously described in the specification. The seryl-tRNA synthetase modulator is, in a preferred embodiment, a seryl-tRNA synthetase inhibitor. The pharmaceutical composition is one of the same that previously described in the specification.

In a fifth aspect, the invention relates to a liquid for ex *vivo* organ preservation comprising a seryl-tRNA synthetase modulator or a pharmaceutical composition comprising at least one seryl-tRNA synthetase modulator and a pharmaceutically acceptable carrier. The seryl-tRNA synthetase modulator is one of the same that previously described in the specification. The seryl-tRNA synthetase modulator is, in a preferred embodiment, a seryl-tRNA synthetase inhibitor. The pharmaceutical composition is one of the same that previously described in the specification.

In a sixth aspect, the invention relates to a method for preserving or treating organs in their viable states in brain-dead patients or cadavers, for organ harvesting, whereby the harvested organs are suitable for transplantation.

A used herein, a "brain-dead patient" is a patient who has a complete loss of brain function. In such a case, the continuing of all or some vital organs functions in the patient is, at least to some extent, maintained through mechanical ventilation and other life-support features.

In particular, the method involves the administration of a seryl-tRNA synthetase modulator or a pharmaceutical composition comprising at least one seryl-tRNA synthetase modulator and a pharmaceutically acceptable carrier, to the brain-dead patient or the cadaver. The seryl-tRNA synthetase modulator is one of the same that previously described in the specification. The seryl-tRNA synthetase modulator is, in a preferred embodiment, a seryl-tRNA synthetase inhibitor. The pharmaceutical composition is one of the same that previously described in the specification. In a preferred embodiment, the seryl-tRNA synthetase modulator or the pharmaceutical composition is administered by intraperitoneal or intravenous route.

In a seventh aspect, the invention relates to a method for preserving or treating an harvested organ in their viable states. In particular, the method comprise the administration of a seryl-tRNA synthetase modulator or a pharmaceutical composition comprising at least one seryl-tRNA synthetase modulator and a pharmaceutically acceptable carrier by perfusion to said harvested organ.

As used herein, an "harvested organ" is an organ which belongs to a brain-dead patient or a cadaver. An harvested organ according to the invention is suitable for transplantation.

The seryl-tRNA synthetase modulator is one of the same that previously described in the specification. The seryl-tRNA synthetase modulator is, in a preferred embodiment, a seryl-tRNA synthetase inhibitor. The pharmaceutical composition is one of the same that previously described in the specification.

### MATERIALS AND METHODS:

### 1- Cell culture and experimental conditions

### Cell lines

HT22 mouse hippocampal neuronal cells were cultured in Dulbecco's modified Eagle's medium (DMEM; Gibco) supplemented with 10% FBS (Gibco), 100U/ml streptomycin, 100U/ml penicillin at 37 °C in 5% CO₂/95% air. Renal PCT cells were obtained from primary cultures of murine proximal tubule segments and cultured as previously described (Melis, N. et al. Targeting eIF5A Hypusination Prevents Anoxic Cell Death through Mitochondrial Silencing and Improves Kidney Transplant Outcome. J Am Soc Nephrol 28, 811-822, doi:10.1681/ASN.2016010012 (2017)). Hypoxic or anoxic conditions were created by maintaining cell cultures at 37°C in a sealed "Bug-Box" anaerobic workstation (Ruskin Technologies). Oxygen Glucose Deprivation (OGD) was induced on HT22 cells placing them in glucose-free Hepes balanced salt solution without glucose (HBSS: NaCl 116mM, KCl 5.4mM, CaCl2 1.8mM, MgSO4 0.8mM, NAH₂PO4 1mM, NaHCO₃ 26.2mM,; pH 7.2-7.4) in an hypoxic chamber filled with 98.5% N2 and 1.5% CO₂ at 37°C for 3h. Upon removal of the cultures from the chamber, the growth medium was changed back to normal medium.

### Cell Viability/Cytotoxicity assay

The Fluorescent LIVE/DEAD® Cell Viability/Cytotoxicity Assay Kit (Invitrogen, France) was used on PCT cells (24-well plates) according to the manufacturer's protocol. Fluorescent micrographs were recorded using an observer D1 microscope (Zeiss).

FACS analysis of HT22. After incubation of the HT22 cells in the different experimental conditions, the cells were incubated with propidium iodide (PI homodimeric, 20 min, 0.04µM). Cells were then trypsinized, washed (HBSS) and red fluorescence intensity was measured by flow cytometric analysis using a FACSCanto II (BD Biosciences) according to the manufacturer specifications. Dead cells that have lost their membrane integrity exhibit a PI-labelled nucleus.

### 2D gel electrophoresis and mass spectrometry analysis.

Trypsinised cells (control and GC7 treated) were homogenised and sonicated in lysis buffer (50 mM Tris, 250 mM mannitol, 1 mM MgCl2, 4% CHAPS, and 50 pg/ml PMSF) and incubated with benzonase (30 U/ml, 60 min, RT). TCA was then added to the protein samples (20% final) and maintained on ice. After 15 min, the samples were centrifuged (13,000 g, 20 min) and the pellets were washed three times with cold acetone. The pellets were then dissolved in rehydration solution (7 M urea, 2 M thiourea, 2% CHAPS, 2% SB3-10, 1% ampholytes pH 3-10, 70 mM DTT and 5 mM Tris). The 2-DE gel procedure, in-gel trypsin digestion, image analysis, MALDI-MS analysis and peptide fingerprinting searches were performed as previously described (Barbey, C. et al. Catabolic pathway of gamma-caprolactone in the biocontrol agent Rhodococcus erythropolis. J Proteome Res 11, 206-216, doi:10.1021/pr200936q (2012)).

### Silencing of serRS.

150,000 PCT cells were seeded in Petri dishes and transfected 24 h later with 50 nM serRS pooled siRNAs (Sigma MISSION®esiRNA targeting mouse serRS: EMU081851) using Lipofectamine™ 3000 transfection reagent (Invitrogen). Cells were exposed to various experimental procedures 24-48 h post-transfection, as described in the text.

### qRT-PCR analysis.

Reverse transcription was performed using 2 µg each RNA sample, M-MLV-RT (Promega) and random primers (250 ng/pL, Roche Diagnostics). Prior to the reaction, genomic DNA contamination was eliminated using DNAse RQ1 (Promega) treatment in the presence of RNAsin (Promega) . Primer sequences were designed using Primer Express software (Applied Biosystems, Courtaboeuf, France) and tested for specificity, efficiency, reproducibility and dynamic range. For the quantitative PCR analyses, 20-µl reactions were prepared using SYBR green master mix (Eurogentec, Angers, France) and 100 nM each primer. Assays were performed using an ABI Prism 7700 real-time PCR machine (PerkinElmer Life and Analytical Sciences, Boston, MA). The expression levels of tested genes were quantified using the comparative ΔCt method with hprt1 as a reference gene. The primer sequences were as follows:
serRS-s: 5'-GTCGGGGCTACACTCCAATC-3'
serRS-as: 5'-TTTCGCTGCCTTTGCCAATC-3'
hprt-s: 5'-GCTGGTGAAAAGGACCTCT-3'
hprt-as: 5'-CACAGGACTAGAACACCTGC-3'

### 2- In vivo model of ischemic stroke

### Animals.

Male 7-week old C57BL/6J mice (Janvier France Breeding) were housed in a temperature- and humidity-controlled animal facility with a 12-h light-dark cycle. Food and water were available ad libitum. All animal care and use were performed with in accordance to the policies of European Community Directive 86/609/EEC. All efforts were made to minimize animal suffering and the number of animals used.

### tMCAo stroke model

tMCAO was performed 30 min after the final injection using a 6-0 coated filament (Doccol), as previously described (Bourourou, M., Heurteaux, C. & Blondeau, N. Alpha-linolenic acid given as enteral or parenteral nutritional intervention against sensorimotor and cognitive deficits in a mouse model of ischemic stroke. Neuropharmacology 108, 60-72, doi:10.1016/j.neuropharm.2016.04.040 (2016)). Control of tMCAo severity and reperfusion was evaluated monitoring the regional cerebral blood flow by laser-Doppler flowmetry. Infarct volume (mm³) was mesured 24 h post-reperfusion on coronal frozen brain sections (20 mm thick) stained by 1% cresyl violet in 0.25% acetic acid using a computer image analysis system.

### serRS inhibiting treatment with serHX.

Animals were randomly assigned to vehicle or serHX groups prepared for 3 experimental protocols. Protocol 1: Vehicle and serHX were administrated intraperitoneally (416 mM, 150µl IP) 48, 24, 2h before a 60min tMCAo. Protocol 2: The second pretreatment protocols consisted in a single IV injection (416 mM, 75µl IP) 2h before 60 min of tMCAo. Portocol 3: the post-treatment consisted in sequential injections 2h, 2d, 4d, 7d, 10d, 14d and 17d after 30 min tMCAo.

### Motor recovery

It was evaluated by the rotarod test as previously described (Bourourou, M., Heurteaux, C. & Blondeau, N. Alpha-linolenic acid given as enteral or parenteral nutritional intervention against sensorimotor and cognitive deficits in a mouse model of ischemic stroke. Neuropharmacology 108, 60-72, doi:10.1016/j.neuropharm.2016.04.040 (2016)). Mice were tested during the first four days post-stroke to detect the effect of SerHX post-treatment on motor deficits. The day before the tMCAo, after 5 min training on the rotarod at a constant speed (4 rpm), a single trial was performed to measure the latency to fall from the rotating rod (acceleration from 4 to 40 rpm over a period of 10 min) to set up the baseline (pre-operative score). The same protocol was applied 2, 3 and 4 days after tMCAo to evaluated the evolution of motor impairments.

### Cognitive recovery

It was evaluated by the Morris water maze test at 14-18 days after tMCAo, in a 120-cm-diameter, 60-cm-high circular swimming pool filled to a depth of 32 cm with 21±2 °C opaque water, as previously described (Bourourou, M., Heurteaux, C. & Blondeau, N. Alpha-linolenic acid given as enteral or parenteral nutritional intervention against sensorimotor and cognitive deficits in a mouse model of ischemic stroke. Neuropharmacology 108, 60-72, doi:10.1016/j.neuropharm.2016.04.040 (2016)). Learning deficits were evaluated measuring the escape latency that corresponds to the time spent to locate the submerged platform (11 cm of diameter) from 14 to 17 days after tMCAo. Spatial memory deficits were evaluated at 18 days after tMCAo by measuring the time spent in the target quadrant when the platform was removed. Swim pattern of the probe test at 18 days was recorded by ANY-maze software (Version 5.6; Stoelting Co., Wood Dale, Il).

### EXAMPLES:

### Example 1: Pharmacological inhibition of seryl-tRNA synthetase (SerRS) protein prevents anoxia-induced cell death

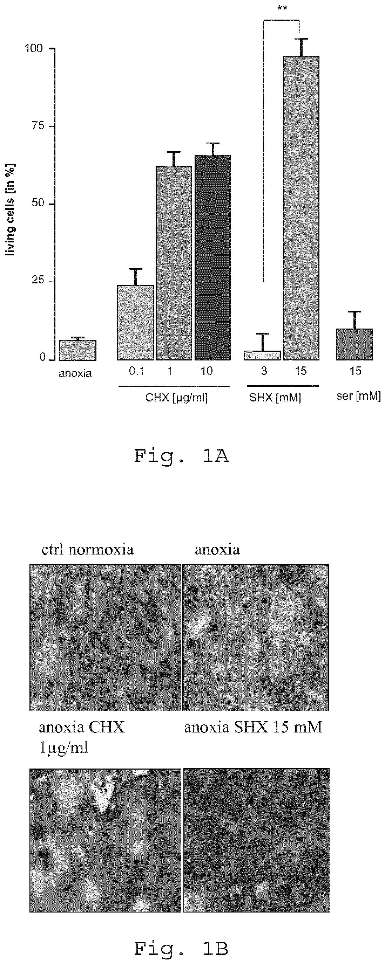

Fig.1A shows the quantification of kidney cells viability under anoxia (24 h, <0.1% O₂) conditions, wherein said cells are prior subjected to inhibitors including cycloheximide and serine hydroxamate. 1 to 10 pg/ml cycloheximide (CHX) increased renal cell survival after 24 h of anoxia. In such a case, cycloheximide which is a global inhibitor of the translational machinery displays 64% of living kidney cells. The serine hydroxamate (SHX) inhibitor which is a specific SerRS inhibitor displays 95% of living kidney cells after a 24h ischemic episode The serine is an natural agonist of the SerRS, and it is used as negative control and had no effect. Values are expressed as the mean ± SEM for 600 cells per well and represent 3-12 replicates per condition (**p<0.01, one-way ANOVA).

Fig.1B shows representative fluorescent images of PCT cells exposed to normoxia, anoxia (O₂< 0,1%), cycloheximide (CHX) or serine hydroxamate (SHX). Living cells appeared in green (calcein-AM) whereas dead cells appeared in red (homodimeric BET). SHX inhibitor at 15 mM displays an highly surviving rate of kidney cells subjected to anoxia condition.

### Example 2: RNAi knockdown (siRNA) of SerRS prevents anoxia-induced cell death

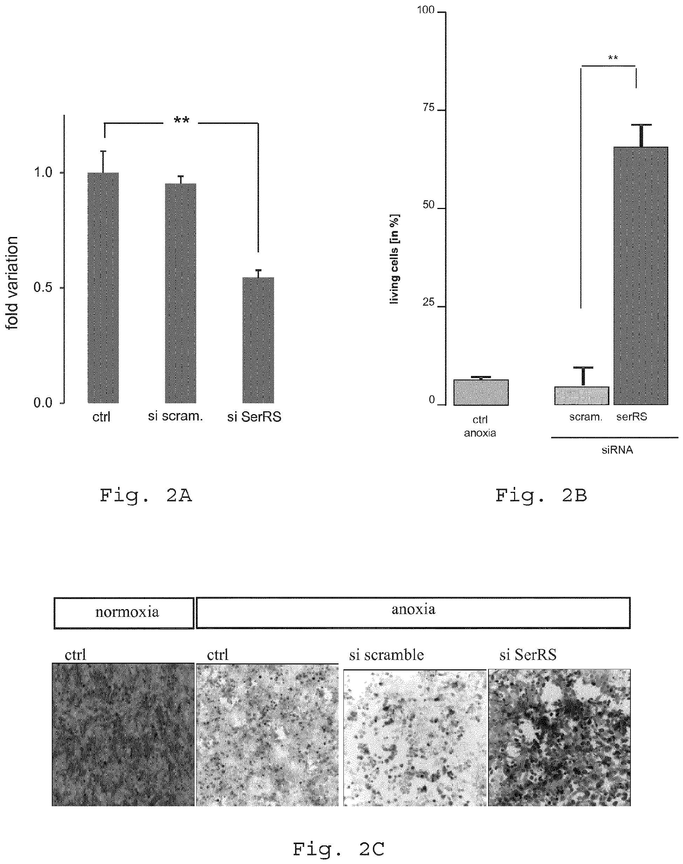

Fig.2A shows mRNA expression levels of the serRS gene in control cells and in cells transfected with non-targeting (siScramble) or serRS pooled siRNAs. qRT-PCR experiments were performed on mRNA extracts obtained 48 h after transfection. siRNA interferes with the expression of specific genes with complementary nucleotide sequences by degrading mRNA after transcription preventing translation. In other words, siRNA causes gene silencing through repression of transcription. The "scramble" siRNA (siScramble) is used as a negative control strategy. siScramble has the same nucleotide composition, but not the same sequence, as the test siRNA. When their sequences are confirmed to not target a known gene or have an miRNA seed region match with a known gene, these siRNA are appropriate as a negative control siRNAs.

Regarding Fig.2A, it is shown cells transfected with non-targeting (siScramble) siRNAs displays almost the same result than the control cells. The siScramble does not inhibit mRNA encoding serRS because it is non-specific of SerRS gene. However, the use of siRNA targeting the SerRS gene inhibits by the serRS encoding mRNA synthesis by 50%.

Fig.2B shows the quantification of kidney cells viability under anoxia (24 h, O₂<0.1%) after mRNA silencing of serRS (siRNA, 48 h). While a scrambled control siRNA (scram) displays no effect, siRNA targeting SerRS gene allows to protect 65% of kidney cells. Finally, silencing the SerRS gene leads to protect the kidney cells when said cells are subjected to ischemic episode.

Fig.2C shows a representative fluorescent images of PCT kidney cells exposed to normoxia or anoxia and which are transfected with serRS or non-targeting control pooled siRNAs (48 h, scrambled siRNA) and maintained for 24 h under anoxia. Living cells appeared in green (calcein-AM) whereas dead cells appeared in red (homodimeric BET). si-RNA targeting SerRS gene displays an highly surviving rate of kidney cells subjected to anoxia condition. Altogether these data confirm that inhibition of seryl-tRNA synthetase increase cell tolerance to anoxia.

### Example 3: Pharmacological inhibition serRS prevents neuronal cell death in vitro and IP and IV pretreatment with serHX inhibition of serRS protects brain against transit focal cerebral ischemia

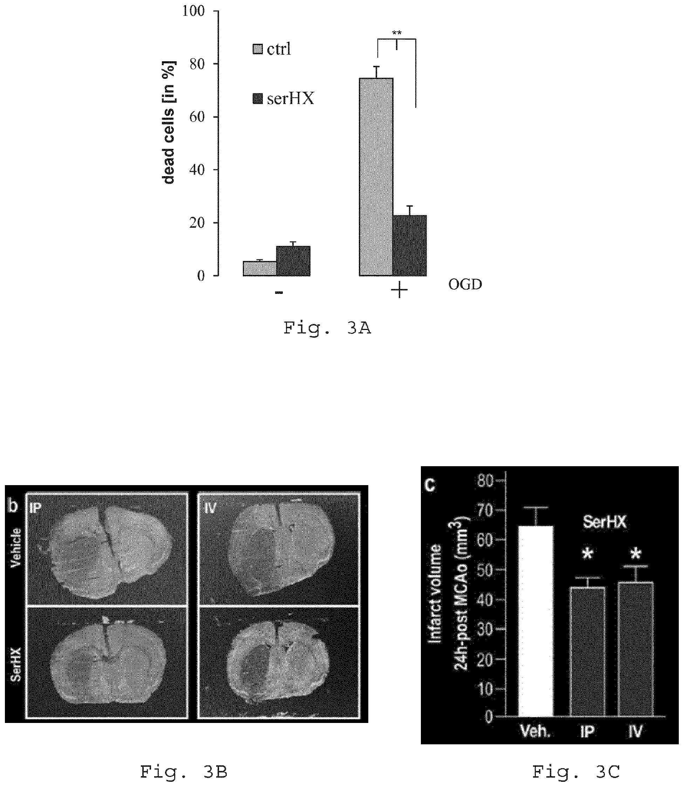

Fig.3A shows a quantification of cell death in HT22 cells (an immortalized mouse neuronal cell line) in control conditions (-) or 24 hours after an Oxygen Glucose Deprivation (OGD) challenge (+) during 3h. 15 mM of serine hydroxamate (serHX) were added 3 hours before the OGD. The use of serHX pretreatment leads to a protection of 78% of cells during a 3h OGD.

Fig.3B shows representative cresyl violet staining showing the protective effect of three successive intraperitoneal (IP) injections of 150µl serHX at 416mM in saline solution 48, 24 and 2h (416 mM, 150µl, daily for 3 days) or a single intravenous (IV) injection (416 mM, 75µl) 2h before intraluminal occlusion of the right middle cerebral artery in mice (tMCAo). In these conditions and taking into account the liquid volume of the mice in which the serHX can diffuse the estimated circulating concentration of serHX is around the one used in *in vitro* assays. Again, serHX reduced cerebral infarction as shown by the histological examination of brain sections 24h after tMCAo (see also Fig.3C). Moreover a single IV injection of SerHX provided 2h before reperfusion induced a similar magnitude of brain protection (see also Fig.3C).

Fig.3C shows a quantitation of the infarct volume confirming the reduction of the ischemic lesion by SerHX pretreatment. Compared with the vehicle group, the infarct volumes were significantly reduced by 30% in the 15 mM serHX group. Values are expressed as the mean ± SEM, n=3-11, *p<0.05, **p<0.01 one way ANOVA.

### Example 4: SerHX post-treatment improves motor and cognitive recovery post-stroke

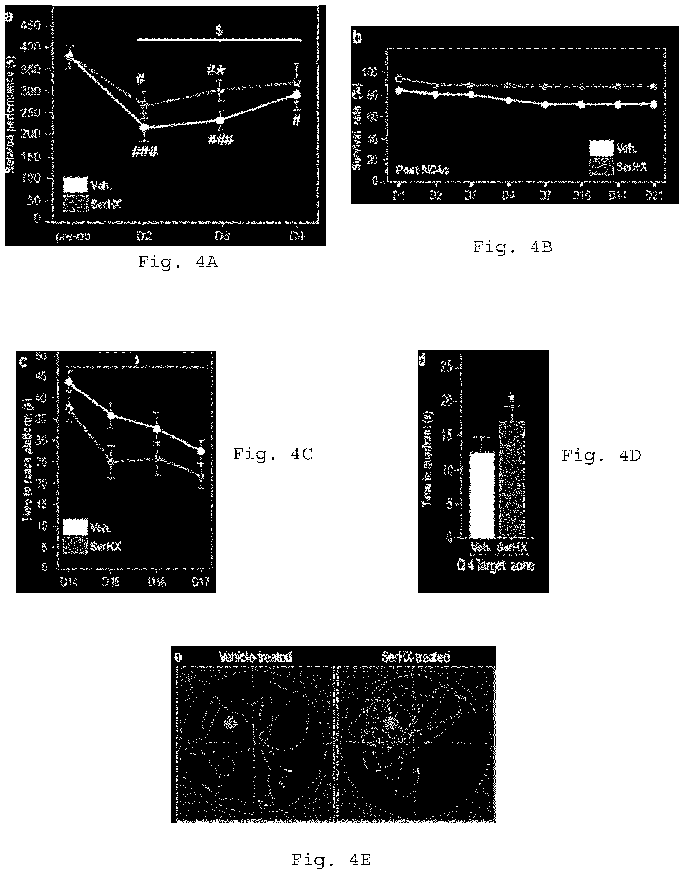

Fig.4A shows the Rotarod test which is performed on mice the first four days post-stroke to detect the effect of SerHX post-treatment on motor deficits. In such a test that is used to evaluate fore and hind limb motor coordination and balance by measuring the duration of animals remaining on the accelerating rotating rod, SerHX post-treatment (IP injection 2 hours and 2 days post-transient Focal Cerebral Ischemia (tFCI)) improved post-stroke recovery of motor functions. The post-ischemic rotarod performance of serHX-treated mice is significantly improved during the four days of recovery (2W ANOVA: effect of group: F = 4.07, $p = 0.048). Data are represented by mean ± SEM (n=13 for Veh. and 9 for serHX at each time point). #p<0.05; ###p<0.001 versus pre-ischemic performance; *p<0,05 different from vehicle-treated control.

Fig.4B shows the long-term survival rate of mice after 30 min of tMCAo. Such a study presents similar results between serHX and vehicle-treated groups (n = 30 for Veh. and 20 for serHX groups).

Fig.4C/D/E are related to the Morris Water-Maze test. The inhibiting seryl-tRNA synthetase with SerHX post-treatment on spatial learning (Fig.4D) and memory (Fig.4E) using the Morris Water-Maze test at 2 weeks after tMCAo is studied.

In particular, Fig.4C displays that during the learning period, serHX post-treatment improves latency to find the hidden platform (2W ANOVA: effect of group: F = 3.95, $p = 0.05021).

Regarding Fig.4D and Fig.4E, the results showed that serHX treatment, even added after reperfusion, significantly improved learning (reducing latency to the hidden platform, Fig.4D, (p<0.05) (n=14 for Veh. and 9 for serHX groups), spatial memory (increasing the time spent in the target quadrant, Fig.4E) and searching behaviors of the platform that has been removed for the test (Fig.4D).

### CONCLUSION:

Altogether, these results not only describe that serRS downregulation and/or reduction of its activity is a crucial step in the ischemia tolerance, but also demonstrate the *in vitro* and *in vivo* relevance of serRS targeting as novel therapeutic opportunity against ischemic conditions and stroke.

These results are obtained on kidney and neuronal living cell, but they can be extend to other kinds of cells because SerRS are present in any sort of mammalian cells that all need oxygen to survive.

## Claims

1. Seryl-tRNA synthetase modulator for use in preventing and/or treating disorders related to hypoxic conditions and/or ischemia injury in a mammal in a need thereof.

2. Seryl-tRNA synthetase modulator for use according to claim 1, wherein the modulator is an inhibitor.

3. Seryl-tRNA synthetase modulator for use according to any one of the preceding claims, wherein the disorders related to hypoxic conditions are selected from the group consisting of kidney failure, heart failure, neurological disorders, stroke, heart attack, atrial fibrillation, angiopathy, peripheral and central microangiopathy, thrombosis, infarction, pulmonary embolism, ischemic colitis and mesenteric ischemia, and organ dysfunction following a transplantation procedure said organs being selected from liver, bowel, heart, and lung.

4. Seryl-tRNA synthetase modulator for use according to any one of the preceding claims, wherein the modulator is selected from the group consisting of serine hydroxamate, serine analogs, non-metabolisable serine derivatives, compound SB-217452, molecules able to specifically bind seryl tRNA synthetase in a competitive, uncompetitive, or noncompetitive way and siRNA targeting seryl-tRNA synthetase gene.

5. Seryl-tRNA synthetase modulator for use according to any one of the preceding claims, wherein the modulator is administered for enhancing hypoxia tolerance of mammal cells subject to hypoxic conditions and/or ischemia injury.

6. Seryl-tRNA synthetase modulator for use according to any one of the preceding claims, wherein the seryl-tRNA synthetase is in its mitochondrial form or its cytoplasmic form, preferably in its cytoplasmic form.

7. Seryl-tRNA synthetase modulator for use according to any one of the preceding claims, wherein it is administered by parenteral delivery and enteral delivery including per os, nasogastric, percutaneous endoscopic gastrostomy tubing, aerosol, intraperitoneal and intravenous route.

8. Seryl-tRNA synthetase modulator for use according to any one of the preceding claims, wherein it is administered by one single injection, by sequential injections or by continuous infusion preceding or following the disorders related to hypoxic conditions and/or ischemia injury.

9. A pharmaceutical composition for use in preventing and/or treating disorders as described in any one of claims 1 or 3, said composition comprising at least one seryl-tRNA synthetase modulator as described in any one of claims 1, 2 or 4 and a pharmaceutically acceptable carrier.

10. The pharmaceutical composition for use according to claim 9, wherein said composition is administered by parenteral delivery and enteral delivery including, per os, nasogastric, percutaneous endoscopic gastrostomy tubing, aerosol, intraperitoneal and intravenous route.

11. The pharmaceutical composition for use according to any one of claims 9 to 10, wherein it is administered by one single injection, by sequential injections or by continuous infusion preceding or following the disorders related to hypoxic conditions and/or ischemia injury.

12. Use of a seryl-tRNA synthetase modulator as described in any one of claims 1, 2 or 4, or a pharmaceutical composition as described in any one of claims 9 as cytoprotective agent in an ex *vivo* organ perfusion and preservation fluid.

13. A seryl-tRNA synthetase modulator as described in any one of claims 1, 2 or 4, or a pharmaceutical composition as described in any one of claims 9, for reducing the oxygen demand of a mammal, preferably a human.

14. A method for preserving or treating organs in their viable states in a brain-dead patient or cadaver, wherein a seryl-tRNA synthetase modulator as described in any one of claims 1, 2 or 4, or a pharmaceutical composition as described in claim 9, is a administered to the brain-dead patient or the cadaver.

15. A method for preserving or treating an harvested organ in their viable states wherein a seryl-tRNA synthetase modulator as described in any one of claims 1, 2 or 4, or a pharmaceutical composition as described in claim 9, is administered by perfusion to said organ.
